# FASCICULE DE BREVET EUROPEEN

(11) **EP 2 198 917 B1**
(45) Date de publication et mention de la délivrance du brevet: **30.03.2016**
(21) Numéro de dépôt: 09173448.3
(22) Date de dépôt: 19.10.2009
(51) Int. Cl.: A61N 1/39, A61N 1/08, A61N 1/37

(54) **Générateur pour prothèse cardiaque implantable, comprenant des moyens de mise en sécurité lors d'un examen IRM**
Generator für implantierbarer Herzschrittmacher, die Mittel zur Sicherung während einer Kernspintomographie umfasst
Generator for an implantable pacemaker, comprising means for commuting in safety mode during MRI tests

(30) Priorité: 19.12.2008 FR 0807148
(43) Date de publication de la demande: 23.06.2010
(73) Titulaire: ELA MEDICAL, 92541 Montrouge (FR)
(72) Inventeur: Legay, Thierry, 91640 Fonteny Les Briis (FR)
(74) Mandataire: Dupuis-Latour, Dominique

(56) Documents cités:
- EP-A1- 1 632 265
- WO-A1-2008/112485
- WO-A2-03/063946
- WO-A2-2008/036865
- US-A1- 2007 088 416
- US-A1- 2007 255 332
- US-A1- 2008 221 638

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Ces dispositifs comportent un boîtier généralement désigné "générateur", raccordé électriquement et mécaniquement à une ou plusieurs "sondes" munie(s) d'électrodes destinées à venir en contact avec le myocarde sur des sites de recueil des potentiels électriques et/ou d'application des impulsions de stimulation. Ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

L'invention concerne plus particulièrement les techniques permettant de sécuriser ces dispositifs (générateurs et leurs sondes associées) lorsque le porteur doit être soumis à un examen par imagerie par résonance magnétique (IRM ou MRI).

En effet, un examen IRM est aujourd'hui contre-indiqué aux patients porteurs d'un stimulateur ou défibrillateur cardiaque. Plusieurs types de problèmes en sont la cause :
- un échauffement à proximité des électrodes reliant le générateur au coeur du patient ;
- les forces et couples d'attraction exercés sur le dispositif plongé dans le champ magnétique statique très élevé de l'appareil IRM ;
- un comportement imprédictible du dispositif lui-même, du fait de l'exposition à ces champs magnétiques extrêmes.

La présente invention a pour objet d'apporter une solution au premier type de problème.

Le problème d'échauffement apparaît surtout au voisinage des électrodes des sondes qui sont connectées au générateur. En effet les sondes, placées dans l'imageur IRM, se comportent comme des antennes et captent le champ radiofréquence (RF) émis par l'imageur. La fréquence de ce champ RF est égale à la fréquence de Larmor des protons, soit f = 42,56 x B₀, où B₀, en Tesla, est l'induction statique caractéristique de l'imageur. Pour des inductions statiques typiques B₀ de 1,5 T ou 3 T, les fréquences RF générées corrélativement par l'imageur sont d'environ 64 MHz et 128 MHz, respectivement.

Les sondes plongées dans ce champ RF voient dès lors circuler dans leurs conducteurs des courants induits provoquant autour des électrodes en contact avec le sang un échauffement des tissus environnants. En effet, l'échauffement au niveau des électrodes étant proportionnel à la densité de courant circulant dans celles-ci, plus la surface de l'électrode est faible, plus la densité de courant est grande et donc plus l'échauffement des tissus environnants sera élevé.

En pratique, selon la configuration relative du générateur, des sondes et de l'imageur IRM, les élévations de température constatées expérimentalement atteignent typiquement 8°C (pour des électrodes en carbone) à 12°C (pour des électrodes en métal), parfois même jusqu'à 30°C.

Or l'élévation de température ne doit pas être supérieure à ce qui est spécifié dans la norme EN 45502-1 et ses dérivés, soit moins que 2°C. En effet, à partir de 4°C survient une mort cellulaire locale qui a pour effet immédiat, entre autres, de modifier les seuils de détection et de stimulation de manière irréversible.

Il est certes possible, comme le décrit notamment le US 2007/0255332 A1, de prévoir un mode de mise en sécurité IRM dans lequel toute stimulation est inhibée, et dans lequel un circuit de protection prévu au niveau du connecteur du boîtier isole les conducteurs des circuits du générateur, et couple tous ces conducteurs avec la masse du boîtier via des traversées capacitives pour empêcher la circulation de courants parasites induits.

Mais cette manière de procéder empêche le dispositif de rester fonctionnel pendant la durée de l'examen.

Or cet examen peut se prolonger plusieurs minutes, et il est pour cette raison éminemment souhaitable que le dispositif puisse continuer à assurer sans discontinuité les fonctions de détection des potentiels de dépolarisation et de stimulation éventuelle du myocarde (pendant la durée de l'examen IRM, le dispositif est toutefois basculé dans un mode particulier de fonctionnement sécurisé, désactivant notamment les circuits sensibles aux champs magnétiques élevés tels que les circuits de télémétrie RF et les alimentations à découpage).

Il n'est donc pas suffisant, en pratique, de simplement déconnecter tous les conducteurs de sonde et/ou les mettre à la masse pendant la durée de l'examen IRM, pour éviter la formation de courants induits.

Pour réduire la circulation du courant dans les conducteurs de sonde, il a été proposé de mettre en série avec ces conducteurs, sur le chemin des courants induits, des filtres s'opposant au passage du courant. Ces filtres peuvent être constitués d'une simple inductance, toutefois l'atténuation, si elle est sensible, n'est généralement pas suffisante. Il a également été proposé d'intercaler dans la boucle de courant un circuit résonant de type circuit bouchon, accordé sur la fréquence RF générée par l'imageur. Mais cette solution présente l'inconvénient de nécessiter autant de filtres que de types d'imageurs différents (64 MHz, 128 MHz, ...) dans la mesure où la fréquence caractéristique n'est pas toujours la même d'un appareil à l'autre, comme on l'a expliqué plus haut. Ces techniques sont notamment exposées dans le US 2007/0088416 A1.

Le point de départ de l'invention consiste à rechercher une protection d'une autre nature : au lieu de limiter la circulation du courant dans les conducteurs de sonde, l'invention propose d'éviter, au moins de réduire, l'exposition de ces conducteurs aux champs RF générés par l'imageur IRM, en mettant en oeuvre une technique de blindage empêchant l'induction de ce courant dans les conducteurs.

L'utilisation de conducteurs blindés en lieu et place des conducteurs existants serait certes possible, mais impliquerait une nouvelle conception des sondes, et ne serait en tout état de cause pas applicable aux sondes déjà implantées.

La solution proposée par l'invention réside dans la constatation du fait qu'avec les sondes de type bipolaire (dorénavant très répandues), ces sondes comportent deux conducteurs - l'un relié à l'électrode distale de faible surface en contact direct avec le myocarde, l'autre à l'électrode proximale plus étendue, qui reste flottante dans le coeur.

Cette solution consiste, essentiellement, à réaliser pendant la durée de l'examen IRM une configuration particulière de détection et de stimulation du myocarde, dans laquelle seul le conducteur relié à l'électrode distale est utilisé fonctionnellement pour la détection/stimulation, l'autre conducteur étant maintenu de façon permanente au même potentiel que le boîtier métallique du générateur, lui-même relié à la masse électrique du dispositif.

La sonde reste ainsi fonctionnelle pour la détection/stimulation en tant que sonde unipolaire, l'autre conducteur étant utilisé temporairement comme conducteur de blindage.

Plus précisément, l'invention concerne un générateur pour dispositif médical actif implantable du type prothèse cardiaque de stimulation, resynchronisation et/ou défibrillation du type générique divulgué par le US 2007/0255332 A1 précité, c'est-à-dire constitué d'un générateur couplé à une sonde de détection/stimulation où :
- la sonde comporte : au moins deux électrodes côté distal ; un connecteur côté proximal ; et au moins deux conducteurs s'étendant sur la longueur de la sonde de son extrémité distale à son extrémité proximale pour relier les électrodes à des bornes respectives du connecteur de la sonde.
- le générateur comporte : un boîtier métallique ; un circuit électronique de détection/stimulation logé dans ce boîtier ; un connecteur comprenant des bornes de liaison connectées au circuit électronique de détection/stimulation et aptes à être couplées à des bornes respectives du connecteur de la sonde ; et des moyens de mise en sécurité IRM, aptes à placer le circuit du générateur dans une configuration protégée à l'encontre des effets délétères d'une exposition de la sonde aux champs magnétiques susceptibles d'être rencontrés lors d'un examen IRM d'imagerie par résonance magnétique.

De façon caractéristique de l'invention, les moyens de mise en sécurité IRM du générateur comprennent des moyens pour mettre temporairement l'une des bornes de liaison du connecteur au potentiel du boîtier métallique, l'(les) autre(s) borne(s) de liaison restant connectée(s) au circuit électronique de détection/stimulation.

La sonde est de préférence une sonde de type coaxial comprenant un conducteur interne axial relié à une électrode distale, et un conducteur externe relié à une électrode proximale. Dans ce cas, les moyens de mise en sécurité IRM comprennent des moyens pour mettre temporairement au potentiel du boîtier métallique la borne de liaison apte à être couplée au conducteur externe de la sonde, et maintenir connectée au circuit électronique de détection/stimulation la borne de liaison apte à être couplée au conducteur interne de la sonde.

Mais la sonde peut aussi être de type coradial comprenant deux conducteurs externes distincts reliés à deux électrodes respectives. Dans ce cas, les moyens de mise èn sécurité IRM comprennent des moyens pour mettre temporairement au potentiel du boîtier métallique la borne de liaison apte à être couplée à l'un des conducteurs externe de la sonde, et maintenir connectée au circuit électronique de détection/stimulation la borne de liaison apte à être couplée à l'autre conducteur externe de la sonde. L'invention couvre également, un procédé de protection d'un dispositif tel que ci-dessus, ce procédé comprenant une étape de mise en sécurité consistant à mettre temporairement l'une des bornes de liaison du connecteur du générateur au potentiel du boîtier métallique, et à laisser en même temps l'(les) autre(s) borne(s) de liaison connectée(s) au circuit de détection/stimulation.

Le procédé comprend avantageusement une détection préalable d'un champ IRM par le générateur, la mise en sécurité n'étant exécutée que sur détection d'un champ IRM.

Le procédé peut en outre prévoir l'interruption forcée de la mise en sécurité après écoulement d'un laps de temps prédéterminé.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une coupe simplifiée d'une sonde coaxiale conventionnelle (utilisée telle quelle par l'invention, non modifiée).
La Figure 2 est un schéma du circuit électrique de détection/stimulation et des liaisons de ce circuit aux électrodes, illustrant la manière dont ce circuit est modifié pour permettre la mise en oeuvre de l'invention.

En ce qui concerne ses aspects logiciels, l'invention peut être mise en oeuvre par une modification matérielle simple et une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'adaptation de ces appareils à la mise en oeuvre des fonctions de l'invention est à la portée de l'homme du métier, et elle ne sera pas décrite en détail.

L'invention peut notamment être appliquée aux dispositifs implantables tels que ceux des familles *Reply* et *Paradym* produits et commercialisés par ELA Médical, Montrouge, France. Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous.

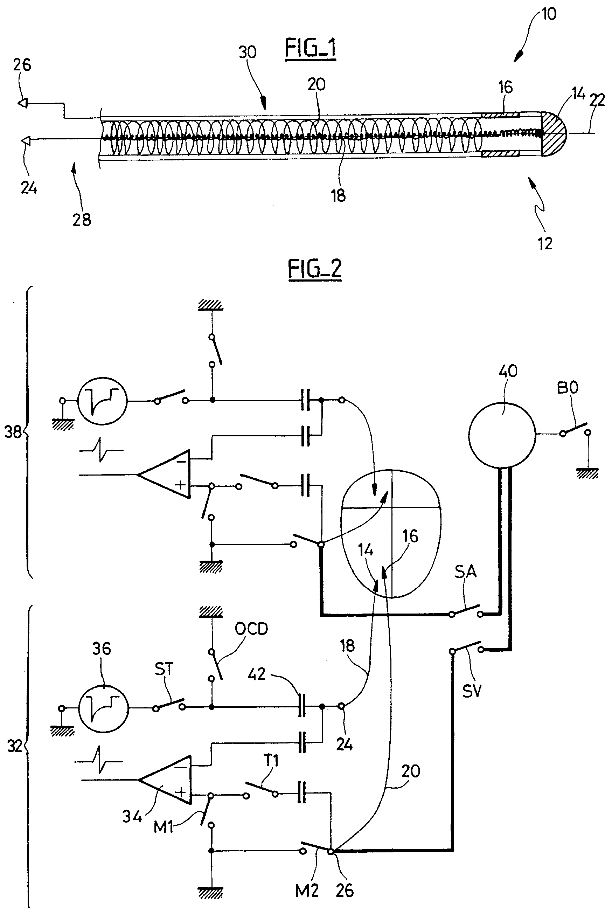

La Figure 1 illustre l'exemple d'une sonde 10 de type "coaxial". À son extrémité distale 12, cette sonde comporte deux électrodes, à savoir une électrode distale d'extrémité ou *"tip"* 14 de faible surface (quelques mm²), destinée à venir directement en contact avec les tissus du myocarde au fond de la cavité auriculaire ou ventriculaire, et une électrode proximale annulaire ou "*ring*" 16, plus étendue (quelques dizaines de mm²), qui reste flottante à l'intérieur de la cavité cardiaque, en interface avec le sang.

L'électrode distale 14 est reliée à un conducteur central 18, tandis que l'électrode proximale est reliée à un conducteur extérieur 20 bobiné en périphérie du corps de la sonde. Ces conducteurs 18, 20 sont bien entendu isolés entre eux et de l'environnement extérieur par des gaines isolantes interne et externe, non représentées.

Le conducteur interne 18 est un conducteur spiralé s'étendant axialement le long de l'axe principal 22 de la sonde, de manière à laisser subsister en partie centrale un espace suffisant pour former une lumière centrale dans laquelle pourra être introduit par exemple un fil de guidage utilisé lors de l'implantation.

Les conducteurs 18, 20 aboutissent à des bornes respectives 24, 26 à l'extrémité proximale de la sonde, ces bornes faisant partie d'un connecteur (non représenté) permettant le couplage mécanique et électrique de la sonde à un générateur du dispositif implanté.

Essentiellement, l'invention propose de mettre et maintenir le conducteur extérieur 20 à la masse (c'est-à-dire au potentiel électrique du boîtier métallique du générateur), afin que ce conducteur 20 joue un rôle de blindage pour le conducteur intérieur 18, sur toute la longueur de la partie centrale 30 de la sonde. Ce blindage fournira une protection analogue à celle obtenue par un câble coaxial du même type que celui largement répandu pour véhiculer les signaux électriques de faible tension. L'invention est également applicable à des sondes de type "coradial", dans lequel les deux conducteurs sont deux conducteurs possédant leur isolation propre, et qui sont spiralés côte à côté autour du même axe principal de la sonde. Dans ce cas, l'effet de blindage est obtenu par la proximité permanente de l'un des conducteurs, porté à la masse, par rapport à l'autre conducteur, relié à l'électrode distale restée fonctionnelle. Dans l'un ou l'autre cas (sonde coaxiale ou coradiale), même si les deux conducteurs sont agencés différemment, ils sont toujours maintenus géométriquement très proches. Le conducteur resté fonctionnel, relié à l'électrode distale, sera protégé par l'autre conducteur et pourra être utilisé pour détecter les ondes cardiaques spontanées et délivrer les impulsions de stimulation. Le blindage par le conducteur proximal relié temporairement à la masse limite le phénomène d'antenne de la sonde placée dans le champ RF, et donc l'induction de courant dans le conducteur distal resté fonctionnel.

La Figure 2 illustre la manière de réaliser ces commutations au niveau du générateur.

On notera qu'il n'est nullement besoin de modifier la sonde pour mettre en oeuvre l'invention, puisque les commutations sont réalisées par le générateur. Ceci présente l'avantage, d'une part, qu'il n'est pas nécessaire de redessiner la sonde et, d'autre part, que l'invention peut être appliquée à des sondes préexistantes, déjà implantées, du simple fait du changement de générateur (le changement du générateur en fin de vie se fait en général sans changement de la sonde).

Le générateur comporte, essentiellement, un étage ventriculaire 32 comprenant un amplificateur de détection ventriculaire 34 et un circuit générateur d'impulsions de stimulation ventriculaire 36. Le générateur peut également comporter un étage auriculaire 38 semblable, que l'on ne décrira pas en détail, dans la mesure où les diverses commutations sont opérées de la même façon, pour aboutir aux mêmes configurations décrites plus bas.

Les circuits de détection/stimulation 34, 36 sont reliés aux électrodes distale 14 et proximale 16 par l'intermédiaire des conducteurs respectifs 18, 20 connectés aux bornes correspondantes 24, 26.

Ces bornes 24, 26 sont couplées aux circuits 34 et 36 par les divers commutateurs M1, M2, T1 et ST. Un commutateur OCD permet également la décharge de la capacité de liaison 42 après délivrance d'une impulsion de stimulation (cet aspect, qui ne fait pas partie de l'invention, ne sera pas décrit plus en détail). Il est également prévu un commutateur B0 permettant de relier sélectivement le boîtier métallique 40 du générateur à la masse électrique des différents circuits logés dans celui-ci.

Pour une configuration de détection bipolaire, l'état des commutateurs est le suivant : B0 fermé, M1 et M2 ouverts, T1 fermé, ST ouvert.

Pour une configuration de détection unipolaire, l'état des commutateurs est le suivant : B0 fermé, M1 fermé, M2, T1 et ST ouverts.

Pour une configuration de stimulation bipolaire, l'impulsion de stimulation est délivrée entre les deux électrodes distale et proximale, l'électrode proximale étant à la masse et le boîtier étant flottant. L'état des commutateurs est alors le suivant : B0 ouvert, M2 et ST fermés pendant la stimulation.

Pour une configuration de stimulation unipolaire, l'impulsion de stimulation est délivrée entre l'électrode distale et le boîtier, l'électrode proximale étant flottante et le boîtier étant relié à la masse. L'état des commutateurs est alors le suivant : B0 fermé, M2 ouvert, ST fermé pendant la durée de la stimulation.

Ces diverses configurations sont en elles-mêmes connues.

L'invention propose de modifier le générateur, et son logiciel de commande, en ajoutant une liaison - en trait épais sur la Figure 2 - entre la borne 26 du générateur (reliée à l'électrode proximale 16) et le boîtier métallique 40 du générateur, cette liaison pouvant être sélectivement fermée par actionnement d'un commutateur SV, pour l'étage ventriculaire. S'il existe un étage de détection/stimulation auriculaire, la même liaison est prévue, avec un commutateur correspondant SA.

L'objet de ces commutateurs SA et SV est de forcer au potentiel de la masse le conducteur de l'électrode proximale (auriculaire ou ventriculaire), pendant toute la durée de l'examen IRM.

Le maintien permanent du potentiel de la masse de l'un des conducteurs de la sonde est une opération non conventionnelle, dans la mesure où, normalement, le générateur gère seulement les configurations classiques unipolaire ou bipolaire de stimulation/détection exposées plus haut.

Les commutateurs SA et SV sont des commutateurs de type relais électronique, MEMS, etc. et sont pilotés par des portes logiques elles-mêmes commandées par le logiciel du générateur.

Dès lors que les conditions pour passer dans un mode sécurisé sont remplies, le logiciel commande l'ensemble des commutateurs du générateur pour obtenir une configuration conforme à l'invention, avec les conducteurs proximaux auriculaire et ventriculaire reliés au boîtier du générateur et à la masse électrique de celui-ci pendant toute la durée de l'examen. L'exposition à des champs magnétiques de type IRM est détectée par diverses techniques en elles-mêmes connues et qui ne seront pas décrites plus en détail, telles que : détection par la saturation du noyau d'une bobine, détection de champ magnétique par un transistor à effet de champ, mesure de la tension recueillie aux bornes d'une antenne de télémétrie, etc., cette détection pouvant être combinée à d'autres critères pris en compte par un algorithme spécifique.

Cette configuration sera maintenue tant que les conditions correspondantes seront réunies, c'est-à-dire tant que le dispositif restera soumis à un champ de forte intensité de type IRM.

Lorsque le dispositif est dans ce mode sécurisé, l'état des commutateurs est le suivant : B0 fermé, M1 fermé, M2 ouvert, T1 ouvert, SV fermé (sauf temporairement pendant la - brève - durée de la stimulation dans une autre cavité (voir ci-dessous)).

Dans ce mode sécurisé selon l'invention, les configurations de stimulation et de détection seront des configurations hybrides, intermédiaires entre les configurations unipolaire et bipolaire classiques. En effet :
- la stimulation aura lieu sur l'électrode distale de la sonde, en référence à la fois au potentiel de l'électrode proximale et au boîtier relié à la masse ;
- quant à la détection des signaux, elle sera du type unipolaire, mais avec un dipôle très court dans la mesure où la masse sera ramenée sur l'électrode proximale.

De préférence, pour éviter un couplage entre les deux chambres ventriculaire et auriculaire, pendant la durée d'application d'une stimulation ventriculaire l'électrode proximale auriculaire n'est pas reliée à la masse (et *vice versa*)*.*

Comme la phase de stimulation est très courte (typiquement 1 ms pour l'impulsion électrique de stimulation, suivie de 14 ms pour la décharge du condensateur de sortie), la brève absence de blindage dans l'étage auriculaire pendant la stimulation ventriculaire (ou inversement) n'a pas d'impact notable sur l'élévation de température des électrodes, qui est un phénomène physique à grande constante de temps par rapport à la durée des phases de stimulation.

Lorsque le dispositif sort du mode sécurisé, c'est-à-dire après la fin de l'examen IRM, la configuration standard de détection/stimulation (décrite plus haut) est rétablie.

Le concept de l'invention est bien entendu généralisable à un générateur conçu pour agir sur un plus grand nombre de chambres, comme avec les dispositifs de type "multisite" utilisés par exemple pour la resynchronisation cardiaque ventriculaire et/ou auriculaire.

On notera que la configuration sécurisée peut être utilisée non seulement pour les examens IRM, mais également comme protection dans divers autres environnements électromagnétiques créés par des appareils médicaux tels que bistouris électriques, dispositifs de stimulation électrique transcutanée des nerfs (TENS), etc. ou encore des équipements de la vie courante tels que portiques antivol, dispositifs de surveillance électrique des articles (EAS), etc.

## Revendications

1. Un générateur pour un dispositif médical actif implantable du type prothèse cardiaque de stimulation, resynchronisation et/ou défibrillation, ledit dispositif comprenant :
- une sonde de détection/stimulation (10) comportant :
- côté distal (12), au moins deux électrodes (14, 16) ;
- côté proximal (28), un connecteur ; et
- au moins deux conducteurs (18, 20) s'étendant sur la longueur (30) de la sonde de son extrémité distale à son extrémité proximale pour relier les électrodes à des bornes respectives (24, 26) du connecteur de la sonde, et
- ledit générateur, apte à être couplé à ladite sonde, comportant :
- un boîtier métallique (40) ;
- un circuit électronique de détection/stimulation (34, 36) logé dans ce boîtier ;
- un connecteur comprenant des bornes de liaison connectées au circuit électronique de détection/stimulation et aptes à être couplées à des bornes respectives du connecteur de la sonde ; et
- des moyens de mise en sécurité IRM, aptes à placer le circuit du générateur dans une configuration protégée à l'encontre des effets délétères d'une exposition de la sonde aux champs magnétiques susceptibles d'être rencontrés lors d'un examen IRM d'imagerie par résonance magnétique,
générateur **caractérisé en ce que** les moyens de mise en sécurité IRM comprennent des moyens (SA, SV) pour mettre temporairement l'une des bornes de liaison (24, 26) du connecteur au potentiel du boîtier métallique (40), l'(les) autre(s) borne(s) de liaison restant connectée(s) au circuit électronique de détection/stimulation.

2. Le générateur de la revendication 1, apte à être couplé à une sonde de type coaxial comprenant un conducteur interne axial (18) relié à une électrode distale (14), et un conducteur externe (20) relié à une électrode proximale (16), ce générateur étant **caractérisé en ce que** les moyens de mise en sécurité IRM comprennent des moyens (SA, SV) pour :
- mettre temporairement au potentiel du boîtier métallique (40) la borne de liaison (26) apte à être couplée au conducteur externe (20) de la sonde, et
- maintenir connectée au circuit électronique de détection/stimulation (34, 36) la borne de liaison (24) apte à être couplée au conducteur interne (18) de la sonde.

3. Le générateur de la revendication 1, apte à être couplé à une sonde de type coradial comprenant deux conducteurs externes distincts reliés à deux électrodes respectives, ce générateur étant **caractérisé en ce que** les moyens de mise en sécurité IRM comprennent des moyens pour :
- mettre temporairement au potentiel du boîtier métallique la borne de liaison apte à être couplée à l'un des conducteurs externe de la sonde, et
- maintenir connectée au circuit électronique de détection/stimulation la borne de liaison apte à être couplée à l'autre conducteur externe de la sonde.

4. Un procédé de protection d'un dispositif médical actif implantable du type prothèse cardiaque de stimulation, resynchronisation et/ou défibrillation à l'encontre à l'encontre des effets délétères d'une exposition aux champs magnétiques susceptibles d'être rencontrés lors d'un examen IRM d'imagerie par résonance magnétique,
- ledit dispositif comprenant une sonde de détection/stimulation (10) et un générateur couplé à ladite sonde,
- ladite sonde comportant au moins deux conducteurs (18, 20) s'étendant sur la longueur (30) de la sonde pour relier des électrodes (14, 16) à des bornes respectives (24, 26) du générateur, et
- ledit générateur comportant un boîtier métallique (40), un circuit électronique de détection/stimulation (34, 36) logé dans ce boîtier, et un connecteur comprenant des bornes de liaison connectées au circuit électronique de détection/stimulation et couplées à la sonde, procédé **caractérisé en ce qu'**il comprend une étape de mise en sécurité, consistant à mettre temporairement l'une des bornes de liaison (24, 26) du connecteur du générateur au potentiel du boîtier métallique (40), et à laisser en même temps l'(les) autre(s) borne(s) de liaison connectée(s) au circuit de détection/stimulation.

5. Le procédé de la revendication 4, comprenant en outre la détection préalable d'un champ IRM par le générateur, et dans lequel l'étape de mise en sécurité n'est exécutée que sur détection d'un champ IRM.

6. Le procédé de la revendication 5, comprenant en outre l'interruption forcée de la mise en sécurité après écoulement d'un laps de temps prédéterminé.

## Patentansprüche

1. Generator für eine implantierbare aktive medizinische Vorrichtung des Typs Herzstimulations-, Resynchronisations- und/oder Defibrillationsprothese, wobei die Vorrichtung Folgendes umfasst:
- eine Erfassungs-/Stimulationssonde (10), die Folgendes umfasst:
• distale Seite (12), mindestens zwei Elektroden (14, 16),
• proximale Seite (28), einen Steckverbinder, und
• mindestens zwei Leiter (18, 20), die sich auf der Länge (30) der Sonde von ihrem distalen Ende zu ihrem proximalen Ende erstrecken, um die Elektroden mit jeweiligen Klemmen (24, 26) des Steckverbinders der Sonde zu verbinden, und
- wobei der Generator geeignet ist, mit der Sonde gekoppelt zu werden, umfassend:
• ein Metallgehäuse (40),
• eine elektronische Erfassungs-/ Stimulationsschaltung (34, 36), die in dem Gehäuse untergebracht ist,
• einen Steckverbinder, der Verbindungsklemmen umfasst, die an die elektronische Erfassungs-/Stimulationsschaltung angeschlossen und geeignet sind, mit jeweiligen Klemmen des Steckverbinders der Sonde gekoppelt zu werden, und
• Mittel zur MRI-Sicherung, die geeignet sind, die Schaltung des Generators in eine gegen die schädlichen Auswirkungen einer Exposition der Sonde gegenüber den Magnetfeldern, die während einer MRI-Magnetresonanztomographie-Untersuchung angetroffen werden können, geschützte Konfiguration zu platzieren,
wobei der Generator **dadurch gekennzeichnet ist, dass** die Mittel zur MRI-Sicherung Mittel (SA, SV) aufweisen, um eine der Verbindungsklemmen (24, 26) des Steckverbinders vorübergehend an das Potenzial des Metallgehäuses (40) zu legen, wobei die andere(n) Verbindungsklemme(n) an die elektronische Erfassungs-/Stimulationsschaltung angeschlossen bleibt (bleiben).

2. Generator nach Anspruch 1, der geeignet ist, um mit einer Sonde des koaxialen Typs gekoppelt zu werden, die einen internen axialen Leiter (18) umfasst, der mit einer distalen Elektrode (14) verbunden ist, und einen externen Leiter (20), der mit einer proximalen Elektrode (16) verbunden ist, wobei dieser Generator **dadurch gekennzeichnet ist, dass** die MRI-Sicherungsmittel Mittel (SA, SV) umfassen, um:
- die Verbindungsklemme (26), die geeignet ist, mit dem externen Leiter (20) der Sonde gekoppelt zu werden, vorübergehend an das Potenzial des Metallgehäuses (40) zu legen, und
- die Verbindungsklemme (24), die geeignet ist, mit dem internen Leiter (18) der Sonde gekoppelt zu werden, mit der elektronischen Erfassungs-/Stimulationsschaltung (34, 36) verbunden zu halten.

3. Generator nach Anspruch 1, der geeignet ist, mit einer Sonde des koradialen Typs gekoppelt zu werden, die zwei getrennte externe Leiter umfasst, die mit zwei jeweiligen Elektroden verbunden sind, wobei der Generator **dadurch gekennzeichnet ist, dass** die MRI-Sicherungsmittel Mittel umfassen, um:
- die Verbindungsklemme, die geeignet ist, mit einem der externen Leiter der Sonde gekoppelt zu werden, vorübergehend an das Potenzial des Metallgehäuses zu legen, und
- die Verbindungsklemme, die geeignet ist, mit dem anderen externen Leiter der Sonde gekoppelt zu werden, mit der elektronischen Erfassungs-/ Stimulationsschaltung verbunden zu halten.

4. Verfahren zum Schützen einer implantierbaren aktiven medizinischen Vorrichtung des Typs Herzstimulations-, Resynchronisations- und/oder Defibrillationsprothese vor den schädlichen Auswirkungen einer Exposition gegenüber den Magnetfeldern, die bei einer MRI-Magnetresonanztomographie-Untersuchung angetroffen werden können,
- wobei die Vorrichtung eine Erfassungs-/ Stimulationssonde (10) und einen Generator, der mit der Sonde gekoppelt ist, umfasst,
- wobei die Sonde mindestens zwei Leiter (18, 20) umfasst, die sich auf der Länge (30) der Sonde erstrecken, um Elektroden (14, 16) mit jeweiligen Klemmen (24, 26) des Generators zu verbinden, und
- wobei der Generator ein metallisches Gehäuse (40), eine elektronische Erfassungs-/ Stimulationsschaltung (34, 36), die in diesem Gehäuse untergebracht ist, und einen Steckverbinder umfasst, der Verbindungsklemmen umfasst, die mit der elektronischen Erfassungs-/Stimulationsschaltung verbunden und mit der Sonde gekoppelt sind,
wobei das Verfahren **dadurch gekennzeichnet ist, dass** es einen Sicherungsschritt umfasst, der darin besteht, eine der Verbindungsklemmen (24, 26) des Steckverbinders des Generators vorübergehend an das Potenzial des Metallgehäuses (40) zu legen und gleichzeitig die andere(n) Verbindungsklemme(n) an die Erfassungs-/Stimulationsschaltung angeschlossen zu lassen.

5. Verfahren nach Anspruch 4, das außerdem das vorausgehende Erfassen eines MRI-Felds durch den Generator umfasst, und wobei der Sicherungsschritt nur beim Erfassen eines MRI-Felds ausgeführt wird.

6. Verfahren nach Anspruch 5, das außerdem die zwangsweise Unterbrechung der Sicherung nach Verstreichen einer vorbestimmten Zeitspanne umfasst.

## Claims

1. A generator for an implantable active medical device such as a cardiac prosthesis for stimulation, resynchronization and/or defribrillation, said device comprising:
- a detection / stimulation lead (10) including:
• on the distal side (12), at least two electrodes (14, 16);
• on the proximal side (28), a connector; and
• at least two conductors (18, 20) extending over the length (30) of the lead from the distal end to the proximal end thereof, to link the electrodes to respective terminals (24, 26) of the lead connector, and
- said generator, adapted to be coupled to said lead, including:
• a metal box (40);
• an electronic detection/stimulation circuit (34, 36) housed in this box;
• a connector comprising link terminals connected to the electronic detection / stimulation circuit and adapted to be coupled to respective terminals of the lead connector; and
• MRI safety setting means, adapted to place the circuit of the generator in a configuration that is protected against the deleterious effects of an exposition of the lead to the magnetic fields liable to be encountered during an MRI magnetic resonance imaging examination,
said generator being **characterized in that** the MRI safety setting means comprise means (SA, SV) for temporarily setting one of the link terminals (24, 26) of the connector to the potential of the metal box (40), the other link terminal(s) remaining connected to the electronic detection/stimulation circuit.

2. The generator of claim 1, adapted to be coupled to a lead of the coaxial type comprising an axial internal conductor (18) linked to a distal electrode (14), and an external conductor (20) linked to a proximal electrode (16), said generator being **characterized in that** the MRI safety setting means comprise means (SA, SV) for:
- temporarily setting to the potential of the metal box (40) the link terminal (26) adapted to be coupled to the external conductor (20) of the lead, and
- keeping connected to the electronic detection/stimulation circuit (34, 36) the link terminal (24) adapted to be coupled to the internal conductor (18) of the lead.

3. The generator of claim 1, adapted to be coupled to a lead of the coradial type comprising two distinct external conductors linked to two respective electrodes, this generator being **characterized in that** the MRI safety setting means comprise means for:
- temporarily setting to the potential of the metal box the link terminal adapted to be coupled to one of the external conductors of the lead, and
- keeping connected to the electronic detection / stimulation circuit the link terminal adapted to be coupled to the other external conductor of the lead.

4. A method for protecting an implantable active medical device such as a cardiac prosthesis for stimulation, resynchronization and/or defribrillation against the deleterious effects of an exposition to the magnetic fields liable to be encountered during an MRI magnetic resonance imaging examination,
- said device comprising a detection/stimulation lead (10) and a generator coupled to said lead,
- said lead comprising at least two conductors (18, 20) extending over the length (30) of the lead to link electrodes (14, 16) to respective terminals (24, 26) of the generator, and
- said generator including a metal box (40), an electronic detection/ stimulation circuit (34, 36) housed in this box, and a connector comprising link terminals connected to the electronic detection / stimulation circuit and coupled to the lead,
said method being **characterized in that** it comprises a safety setting step, consisting in temporarily setting one of the link terminals (24, 26) of the generator connector to the potential of the metal box (40), and to leave at the same time the other link terminal(s) connected to the detection/stimulation circuit.

5. The method of claim 4, further comprising the previous detection of an MRI field by the generator, and wherein the safety setting step is executed only upon detection of an MRI field.

6. The method of claim 5, further comprising the forced stopping of the safety setting step after a predetermined time lapse has passed.
